# EUROPEAN PATENT SPECIFICATION

(11) **EP 1 542 593 B1**
(45) Date of publication and mention of the grant of the patent: **05.12.2007**
(21) Application number: 03754499.6
(22) Date of filing: 11.09.2003
(51) Int. Cl.: A61B 17/02

(54) **SEPTAL PUNCTURE DEVICE**
VORRICHTUNG ZUR PUNKTION EINES SEPTUMS
DISPOSITIF DE PERFORATION SEPTALE

(30) Priority: 23.09.2002 US 412952 P
(43) Date of publication of application: 22.06.2005
(73) Proprietor: NMT Medical, Inc., Boston, MA 02210 (US)
(72) Inventor: CHANDUSZKO, Andrzej, South Boston, MA 02127 (US); CALLAGHAN, David, J., Boston, MA 02125 (US); WIDOMSKI, David, Wakefield, MA 01880 (US)
(74) Representative: Greenwood, John David
(86) International application number: PCT/US2003/028532
(87) International publication number: WO 2004/026147

(56) References cited:
- EP-A- 1 046 375
- US-A1- 2002 010 481
- US-B1- 6 322 548

## Description

### FIELD OF THE INVENTION

The present invention relates generally to apparatus for stabilizing and/or forming openings in tissues, for example, a system that enables a flexible member to position a cutting member relative to a tissue surface such that an incision can be made by the cutting member in the tissue and more particularly, to a septal puncture apparatus including a flexible member and a cutting member that positions the cutting member for introducing a hole in the atrial septum near a patent foramen ovale defect to aid in closure of the defect by implanting a prosthetic occlusion device in a patient or using a remote apparatus for joining tissue (e.g., a remote suturing, stapling, gluing, or tissue welding tool).

### BACKGROUND OF THE INVENTION

Defects in the septum or vessels of the heart take various forms. The defects sometimes exhibit themselves as occlusions in a vessel or as openings in a chamber of the heart, tissue, or vascular wall.

A septum is generally defined as a dividing wall, membrane, or tissue between two or more bodily spaces. A defect sometimes found in the wall of the heart involves an opening or fluid communication between two chambers of the heart, for example, between the atria. One defect is specifically referred to as a patent foramen ovale (PFO). A PFO is anatomically comprised of two layers of partially overlapping but unfused cardiac tissue, forming a tunnel-like hole or opening between the left and right atria. The existence of this defect can place the patient at a high risk of embolic stroke by allowing for blood in the heart to be abnormally shunted between the two atria.

Septal defects are often treated using septal occluders commonly described and shown in U.S. Pat. No. 5,451,235 to Lock, et al., U.S. Pat. No. 5,578,045 to Das, and U.S. Pat, No. 6,214,029 to Thill, et al.. The devices typically include a pair of occlusion members that are conneted to one another. The occluders typically allow for the occlusion members to be positioned on opposing sides of a hole or opening thereby obstruction the flow of blood or plasma through the opening.

Due to the tunnel-like nature of many PFO's, however, occluders often do not sit flush with the septal wall when implanted directly through the defect opening. For this reason, the use of septal puncture has been proposed and clinical success demonstrated in C.B. Ruiz, B.T. Alboltras. S.G. Pophat, The Puncture Technique: A New Method For Transcatheter Closure of Patent Foramen Ovale, Catheter and Cardiovascular Intervention 2001, 53, 369-372 (2001). Septal puncture allows for the creation of an opening through the septal wall in a more desirable location to maximize the likelihood that an occluder will sit flush with the surface of the septal wall overlapping a septal defect, thereby occluding the defect.

A punch-type device for performing a septal puncture procedure is discussed in U.S. Pat. No. 5,403,338 to Milo. However, in septal punctures, the punch or needles used pose a high risk of inadvertent puncture or damage to tissue other than septum primum and is a significant disadvantage of this procedure. For PFO closure, this risk is potentially even higher, due to the fact that there is defective and often thinning septal tissue, which may stretch an even greater amount risking inadvertent puncture of an unintended anatomical structure or suffer trauma during the puncture procedure.

US-B1-6,322,548 discloses a surgical apparatus having the features of the preamble of claim 1. The present invention is characterized by the features of the characterizing portion of claim 1. Optional features are recited in the dependent claims.

The present invention, which is as claimed in the claims, addresses these risks.

The present invention relates to an apparatus for constraining the motion of biological material, such as tissue walls, adjacent vessels or adjacent regions of an organ and forming a hole in the biological material such that access to opposing surfaces of the biological material can be achieved.

It is another aspect of this invention to provide apparatus for inserting and positioning an elongate member and at least one flexible member in the heart. It is another aspect of this invention to provide an apparatus for limiting the motion of the septal wall of a heart using the at least one flexible member coupled to a distal and of delivery member. It is another aspect of this invention to out a hole in the septal wall of the heart while the at least one flexible member limits motion of the septal wall. Subsequent to cutting a hole in the tissue this present invention also contemplates inserting an apparatus for obstructing holes in the septal wall to limit the flow of blood between adjacent sections of the heart.

Embodiments of this and other aspects of the invention can include the following features. The shape of the flexible members can be polygonal, circular, and/or ellipsoidal. The flexible members can include a wire loop. The at least one flexible member can include a section for stiffening the flexible member. In the second extended position, at least one of the plurality of axes defines an angle relative to the longitudinal axis of the elongate member that is between about 0 degrees and about 180 degrees. The at least one flexible member can limit movement of the tissue when the at least one flexible member is in the second extended position. The flexible members can be biased relative to the first elongate member.

A cutting member for producing a hole in the tissue can be included in alternative embodiments of this invention. The cutting member can produce a hole in the tissue an the at least one flexible member limits movement of the tissue. The cutting member can be disposed within a lumen of the first elongate member and can be axially movable within the lumen. The cutting member can cut the tissue upon emerging form an opening in the lumen. The cutting member could be a needle or sharpened wire. An occlusion device can be included in alternative embodiments of the invention for occluding holes in the tissue. The occlusion device can be a septal occluder, suture, staple, or adhesive. A tissue joining apparatus can be included. The tissue joining apparatus can be a tissue welding apparatus. A second elongate member having a lumen can be provided and the first elongate member can axially move the at least one flexible member substantially co-linearly with a first lumen of the second elongate member.

In general, in another aspect, the invention involves an apparatus for producing a hole in a tissue in a patient. The apparatus includes a catheter having a first lumen that has an opening. The apparatus also includes a delivery member that is axially movable within the frist lumen of the catheter. The delivery member has a first distal end that extends from the opening in the catheter and a second lumen that has an opening. The apparatus also includes a cutting member that is axially movable within the second lumen of the delivery member. The cutting member has a second distal end that extends from the opening in the delivery member and a third lumen that has an opening.

The apparatus can include a guidewire that is axially movable within the third lumen of the cutting member. The guidewire has a third distal end that extends from the opening in the cutting member. The apparatus can include a flexible member that has at least a first free end and a second free end. The first free end and the second free end each undergo a first articulation and a second articulation.

In general, in another aspect, the invention involves an apparatus for producing a hole in a tissue in a patient. The apparatus includes an elongate member having a first lumen that has an opening. The apparatus also includes a coil member that has a first portion and a second portion and is axially movable within the lumen of the elongate member. The coil member is sized and shaped for being gradually transferred out of the opening in the elongate member for placement of the first portion of the coil member adjacent to a first side of a tissue in a patient, and for placement of the second portion of the coil member adjacent a second side of the tissue. Embodiments of the invention can include a cutting member that is axially movable within the lumen of the elongate member in which the cutting member has a distal end that extends from the opening in the elongate member.

The foregoing and other objects, aspects, features, and advantages of the invention will become more apparent from the following description and from the claims.

### BRIEF DESCRIPTION OF THE DRAWINGS

In the drawings, like reference characters generally refer to corresponding parts throughout the different views. The drawings are not necessarily to scale, emphasis instead generally being placed on illustrating the principles and concepts of the invention.

FIG. 1 is a schematic side view of a portion of a septal puncture apparatus including a set of flexible members according to an illustrative embodiment of the invention.

FIG. 2A is a schematic side view of a portion of an embodiment of a septal puncture apparatus including a set of flexible members partially extended from an elongate member according to the invention.

FIG. 2B is a schematic side view of the flexible members of FIG. 3A fully extended from the opening in the elongate member.

FIG. 3 is a schematic side view of another embodiment of a set of flexible members according to the invention.

FIG. 4 is a partially broken-away view of a heart depicting a portion of a septal puncture apparatus, according to the invention, on a second side of the septal wall.

FIG. 5A is a cross-sectional view of a septal wall of a heart depicting a set of flexible members located outside an opening in an end of an elongate member, according to an illustrative embodiment of the invention.

FIG. 5B is a cross-sectional view of the flexible members of FIG. 10A in which a portion of the flexible members is located in contact with a first side of a septal wall and another portion of the flexible members is located in proximity to a second side of the septal wall.

FIG. 5C is a cross-sectional view of the flexible members of FIGS. 10A and 10B in which a cutting member is extended from a lumen in the delivery member creating a hole through the septal wall.

FIG. 6 is a schematic side view of a flexible member, a cutting member, and an elongate member according to an illustrative embodiment of the invention.

FIG. 7A is a cross-sectional view of an illustrative embodiment of a flexible member and elongate member of the invention.

FIG. 7B is a cross-sectional view of the elongate member of FIG. 12A depicting inserting an occlusion device into a hole in the septal wall.

FIG. 7C is a cross-sectional view of the occlusion device of FIG. 12B obstructing holes in the septal wall of a patient.

FIG. 8 is an illustration of an exemplary set of flexible members according to an illustrative embodiment of the invention located in proximity to a patent foramen ovale defect.

### DESCRIPTION OF THE INVENTION

The present invention relates to apparatus for stabilizing (e.g., constraining) the motion of biological material, such as tissue walls, and forming a hole in the biological material. In general overview, a system may include an elongate member for positioning and deploying a flexible member for stabilizing tissue. In one embodiment, the system includes a plurality of flexible members, at least one flexible member positionable on a side of the tissue opposite to another flexible member. Optionally, the system may further include a cutting member for cutting a hole in the septal wall of a heart to position and deploy a prosthetic occlusion device to obstruct blood flow through a septal defect of a cardiac septal wall. Optionally, the system may include a tissue joining apparatus for joining tissue in the body of a patient.

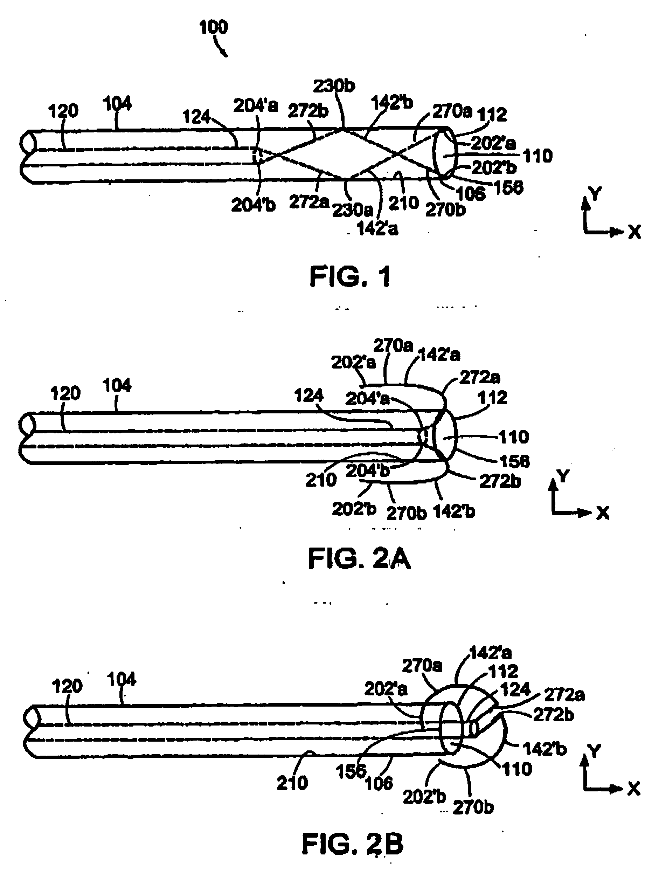

FIG. 1 illustrates a portion of a septal puncture apparatus 100 according to an illustrative embodiment of the invention including exemplary flexible members 142'a and 142'b. Each of the flexible members 142'a and 142'b include a leg such as a wire having a first end 204'a and 204'b, respectively, joined to the distal end 124 of the delivery member 120. Each of the flexible members 142'a and 142'b also have a second distal end 202'a and 202'b, respectively, that is free, i.e., not joined to any other structure of the septal puncture apparatus 100. The longitudinal axis of the flexible members 142'a and 142'b are oriented substantially parallel to the elongate member 104 when the flexible members 142'a and 142'b are located within the lumen 110 of the elongate member 104. The flexible members 142'a and 142'b have a first portion 272a and 272b, respectively and a second portion 270a and 270b, respectively. The flexible members 142'a and 142'b are disposed within the lumen 110 in this contracted position such that the second ends 202'a and 202'b are directed distally towards the opening 112 in the distal end 106 of the elongate member 104.

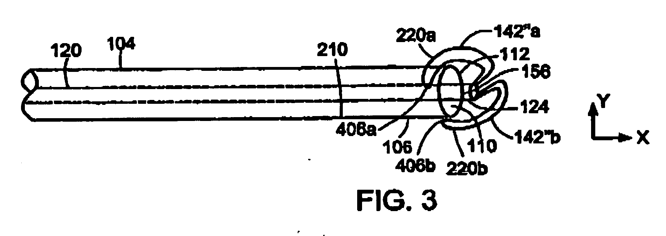

Prior to insertion into the lumen 110, the flexible members 142'a and 142'b are preshaped such that the flexible members 142'a and 142'b will assume a predetermined extended configuration when the flexible members 142'a and 142'b are free from the confines of the lumen 110. The flexible members 142'a and 142'b are freed from the confines of the lumen 110 by moving the flexible members 142'a and 142'b between the contracted position illustrated, for example, in FIG. 2 and an extended position, such as the extended position depicted in FIG. 3B. After insertion into the lumen 110 of the elongate member 104, the flexible members 142'a and 142'b apply a force to an inner surface 210 of the elongate member 104 in a first location 230a and 230b, respectively, on the inner surface 210 of the lumen 110 that the flexible members 142'a and 142'b contact The force applied by the preshaped flexible members 142'a and 142'b to the inner surface 210 is the resultant force associated with the inner surface 210 constraining the shape of the flexible members 142'a and 142'b so they may fit within the lumen 110 of the elongate member 104.

In an embodiment of a septal puncture apparatus, referring now to FIG. 2A, the flexible members 142'a and 142'b are shown partially extended (in comparison with the flexible members 142'a and 142'b in FIG. 1) so the flexible members 142'a and 142'b are still substantially parallel to the longitudinal axis of the elongate body 104. As the delivery member 120 is extended out of the opening 112 of the elongate member 104, the second ends 202'a and 202'b of the flexible members 142'a and 142'b, respectively, undergo an articulation and point, generally, in a proximal direction toward the handle (not shown). In this orientation, the preshaped flexible members 142'a and 142'b apply a force to the rim 156 of the opening 112 in the elongate member 104 because the rim 156 of the opening 112 constrains the shape of the flexible members 142'a and 142'b.

The elongated delivery member 120 is further extended distally, referring now to FIG. 2B, along the lengthwise dimension (in the positive direction along the X-axis) of the lumen 110 until the distal end 124 of the delivery member 120 emerges from the opening 112 of the elongate member 104. The second ends 202'a and 202'b of the exemplary preshaped flexible members 142'a and 142'b, respectively, undergo an additional articulation and as a result point, generally, towards one another. In this extended position, the preshaped flexible members 142'a and 142'b no longer apply a force to the elongate member 104 because the elongate member 104 does not constrain the shape of the flexible members 142'a and 142'b. In this extended position, each of the flexible members 142'a and 142'b is substantially planar in shape. The plane of each of the flexible members 142'a and 142'b define a plurality of axes that lie in the plane. The plurality of axes are non-parallel to (i.e., biased relative to) the longitudinal axis of the elongate member 104. For example, the plurality of axes defined by the planes of the flexible members 142'a and 142'b are positioned at an angle in the range of about 0 degrees to about 180 degrees, preferably, about 90 degrees, relative to the longitudinal axis of the elongate member 104.

In alternative embodiments of the invention, the second ends, for example, the second ends 202'a and 202'b, may have a different diameter than other locations along the length of the flexible elastic members 142'a and 142'b. By way of example, an operator may select an apparatus having flexible members that have second ends 202'a and 202'b having a larger diameter to, for example, reduce trauma to tissue the second ends 202'a and 202'b contact during use. Alternatively, the second ends 202'a and 202'b may have a ball shaped tip.

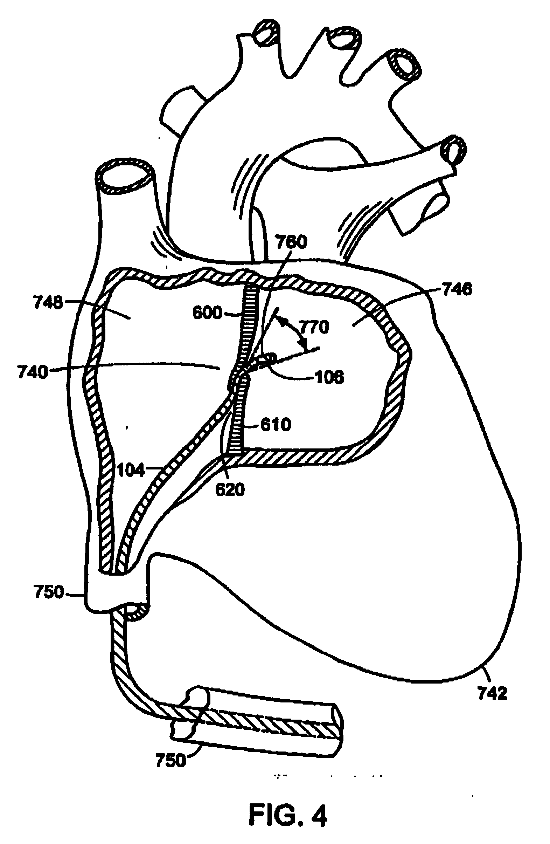

FIG. 3 depicts a portion of a septal puncture apparatus including flexible members according to an alternative illustrative embodiment of the invention. The exemplary flexible members 142"a and 142"b include a first wire loop section 220a and a second loop section 220b, respectively, as illustrated in FIG. 4. The tip 406a and 406b of the loop sections 220a and 220b, respectively, point, generally, towards one another and towards the delivery member 120. Loop sections 220a and 220b may, alternatively, be oriented in a variety of directions (e.g., away from the delivery member 120 or at a 45 degree angle away from the delivery member 120). However, the loops 220a and 220b of the flexible members 142"a and 142"b will, typically, be oriented such that the flexible members 142"a and 142"b including the loop sections 220a and 220b are substantially planar, where the plane defines a plurality of axes lying in the plane. The plurality of axes are non-parallel to (i.e., biased relative to) the longitudinal axis of the elongate member 104 when the flexible members 142"a and 142"b are extended distally from the opening 112 of the elongate member 104. Other embodiments of the flexible member 142"a and 142"b are also contemplated by the invention and are not limited to those illustrated.

In some embodiments of the invention, a septal puncture apparatus can be used to implant alternative occlusion devices (for example, sutures, adhesives, and/or staples) into the body of the patient to join tissue. The occlusion devices may, for example, be a suture of the type described in the co-pending U.S. Provisional Patent Application "Intracardiac Suture" (NMT-022), filed simultaneously herewith. In other embodiment of the invention, a septal puncture apparatus can include a remote tissue joining apparatus (e.g., a tissue welding apparatus) that is used to join tissue in the body of the patient.

Referring now to FIG. 4, there is shown a method for delivery of a prosthetic occluder in the heart of a patient in which an operator introduces an elongate member 104 into the right atrium 748 of a heart 742 through the descending vena cava 750. The elongate member 104 could, for example, be a component of a septal puncture apparatus of the invention.

As further illustrated in FIG. 4, the exemplary elongate member 104 is advanced distally until the distal end 106 of the elongate member 104 passes through a defect 620 (for example, a patent foramen ovale) in the septum 740. The distal end 106 of the elongate member 104 is shown at an angle 770 of about 45 degrees relative to the longitudinal axis of the elongate member 104 due to a bend 760 in the distal end of 106 of the elongate member 104. The bend 760 in the elongate member 104 may be mechanically pre-formed or pre-bent at the angle 770 between about 0 degrees and about 180 degrees prior to insertion of the elongate member into the body. The bend 760 could, alternatively, be accomplished by heating a nickel-titanium material or other shape memory alloy located within the distal end 106 of the elongate member 104. By way of example, the nickel-titanium material might be nickel-titanium wire sold under the product name Nitinol.

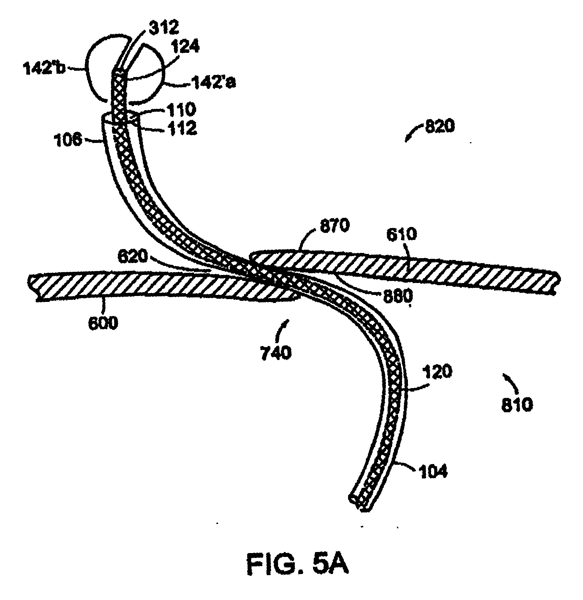

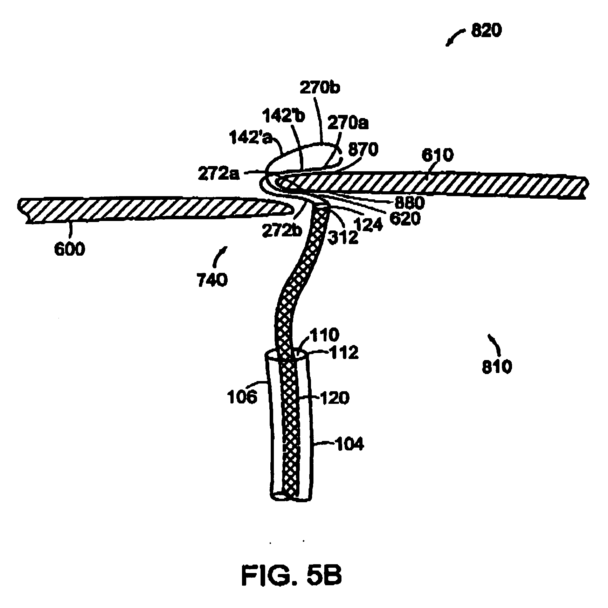

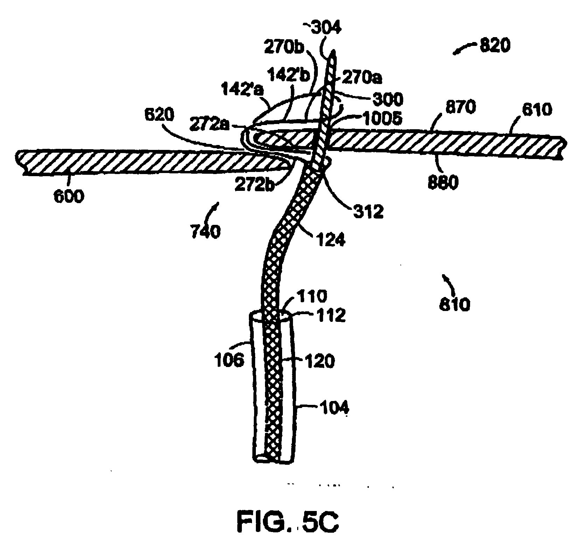

In a particular method of using an apparatus according to the invention for delivering an occlusion device such as a prosthetic septal occluder in the body of a patient, the illustrative septal puncture apparatus in FIGS. 5A, 5B and 5C includes two flexible members 142'a and 142'b coupled to the distal end 124 of the delivery member 120. The flexible members 142'a and 142'b are initially located within the lumen 110 of the elongate member 104 and are in a position in which the flexible members 142'a and 142'b parallel the longitudinal axis of the elongate member 104 as previously described herein. An operator initially guides the distal end of 106 of the elongate member 104 through the defect (hole) 620 such that the distal end 106 is located on a second side 820 (in the left atria of the heart) of the septum secundum 600 and septum primum 610. Now referring to FIG. 5A, operator then extends the flexible members 142'a and 142'b as described herein with respect to, for example, FIGS. 2A and 2B.

With continued reference to FIG. 5A, the elongate member 104 is retracted proximally until the distal end 106 of the elongate member 104 passes back through the defect 620 and is positioned on the first side 810 of the septum 740.

The delivery member 120 is then retracted proximally so the second portions 270a and 270b of the flexible members 142'a and 142'b and the distal end 124 of the delivery member 120 are in close proximity to the defect 620, the septum primum 610, and the septum secundum 600 on the second side 820 of the septum 740.

Now referring to FIG. 5B, as the delivery member 120 is further retracted proximally such that the distal end 124 of the delivery member 120 is withdrawn through the defect 620 until it is in contact with or in close proximity to the first surface 880 of the septum primum 610 on the first side 810 of the septum primum 610. The second portions 270a and 270b of the flexible members 142'a and 142'b are positioned, generally non-parallel to the longitudinal axis of the elongate member 104 and are in physical contact with at least the second surface 870 of the septum primum 610 on the second side 820 of the septum primum 610 and also partially located within the defect 620 in the septum 740. In this exemplary method, the first portions 272a and 272b of the flexible members 142'a and 142'b are located on the first side 810 of the septum 740. Accordingly, the flexible members 142'a and 142'b are sized and shaped for contact with the first side 810 and the second side 820 of the septum 740. The flexible members 142'a and 142'b are thus capable of limiting movement of the septum primum 610. Now referring to FIG. 5C, the cutting member 300 is extended from the opening 312 in the distal end 124 of the delivery member 120. The cutting tip 304 of the cutting member 300 introduces a hole 1005 (tissue opening) through the septum primum 610.

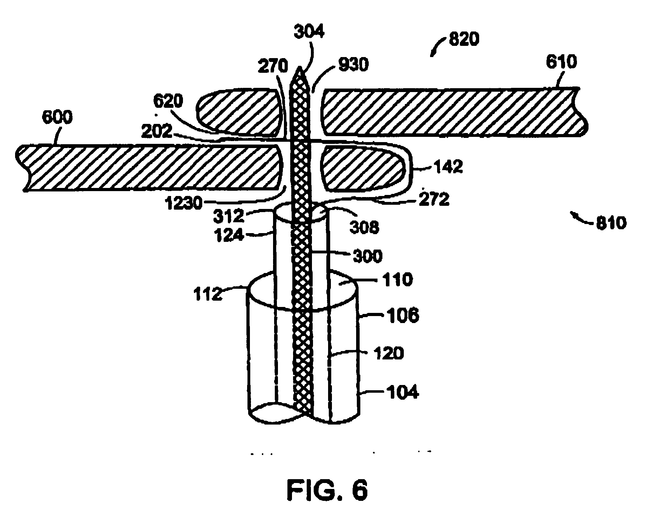

In an alternative method, referring now to FIG. 6, an exemplary flexible member 142 is attached to the distal end 124 of the delivery member 120. The delivery member 120 is located within the lumen 110 of the elongate member 104. The delivery member 120 extends from the opening 112 in the distal end 106 of the elongate member 104. The delivery member 120 and the elongate member 142 are located on the first side 810 of the septum secundum 600. The distal end 124 of the delivery member 120 is located in close proximity to the tissue surface of the septum secundum 600 on the first side 810 of the septum secundum 600. The flexible member 142 extends through the hole 620 between the septum primum 610 and the septum secundum 600 from the first side 810 to the second side 820. The first side 810 of the septum primum 610 opposes the second side 820 of the septum primum 610. The flexible member 142 is positioned so that the second end 202 and second portion 270 of the flexible member 142 are located on the second side 820 of the septum secundum 600 and the first portion 272 of the flexible member 142 is located on the first side 810 of the septum secundum 600. In this configuration, the flexible member 142 is thus capable of limiting movement of the septum secundum 600. In this embodiment only the septum secundum 600 is secured to limit movement In alternative embodiments, however, the septum secundum 600 and/or the septum primum 610 may be secured to limit movement.

In this illustrative method the cutting tip 304 of the cutting member 300 introduces a hole (tissue opening) 1230 through the septum secundum 600 and then a hole (tissue opening) 930 through the septum primum 610. For clarity of illustration purposes, the holes 1230 and 930 are shown larger in diameter than the cutting member 300.

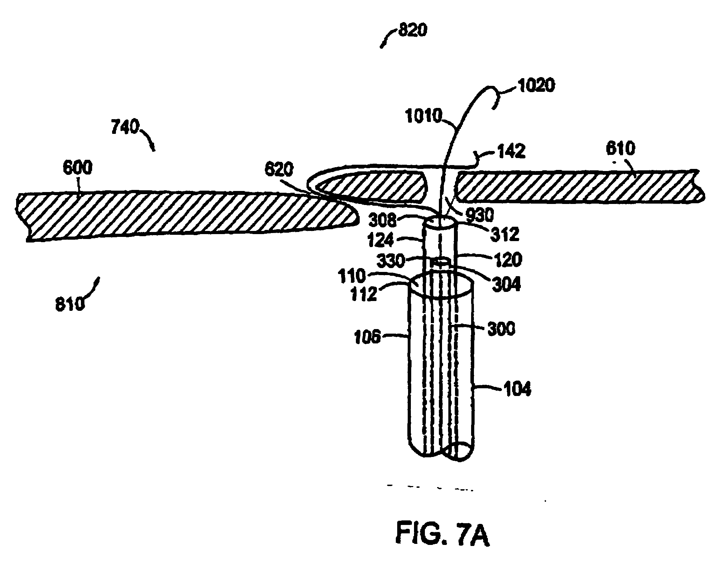

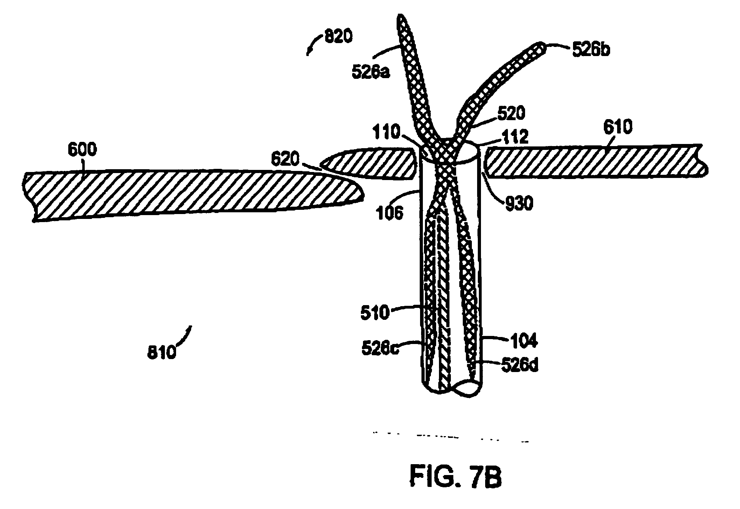

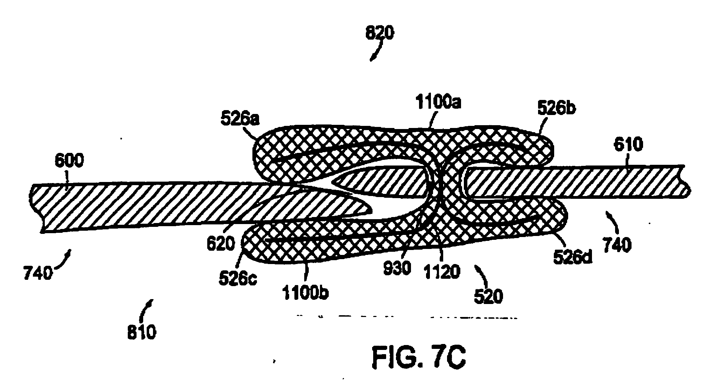

Referring now to FIGS. 7A, 7B and 7C, the illustrative method for delivering an occlusion device using an apparatus according to the invention, the septal puncture apparatus according to the invention includes a guidewire 1010. The illustrative guidewire 1010 is positioned by the operator within a lumen 330 of a cutting member 300. The guidewire 1010 is axially movable within the lumen 330 of the cutting member 300. The cutting member is positioned within and is axially movable within the lumen 308 of the delivery member 120. The flexible member 142 is coupled to the distal end 124 of the delivery member 120. The flexible member 142 extends from the first side 810 of the septum primum 610 through the hole 620 to the second side 820 of the septum primum 610. In this manner, the flexible member 142 limits movement of the septum primum as previously described herein.

A distal end 1020 of the guidewire 1010 extends out of the lumen 330 of the cutting member 300 and the lumen 308 of the delivery member 120 so the distal end 1020 of the guidewire 1010 is located on the second side 820 of the septum primum 610 and the septum secundum 600. By way of example, the guidewire 1010 is used by an operator to aid in guiding the elongate member 104 through the opening 930. The guidewire 1010 is then retracted proximally such that the guidewire 1010 is retracted into the lumen 110 of the elongate member and then withdrawn from the body.

Referring now to FIG. 7B, the delivery member 120 is then retracted proximally such that the flexible member 142 is retracted into the lumen 110 of the elongate member 104. The delivery member 120 is then withdrawn from the body. A dilator member (not shown) is then inserted into the lumen 110 of elongate member 104. The dilator member is used by an operator to, for example, aid in extending the distal end 106 of the elongate member through the opening 930 in the septum primum 610 (as is known to one skilled in the art). The dilator member is then withdrawn proximally through the elongate member 104 and withdrawn from the body. The occlusion device 520 is introduced into the body by the extending member 510 through the elongate member 104. The extending member 510 is coupled to the occlusion device 520. The extending member 510 and the occlusion device 520 pass through the lumen 110 of the elongate member 104 and along the length of the elongate member 104.. The extending member 510 extends axially along the length of the lumen 110 of the elongate member 104 and the occlusion device 520 is passed distally out of the opening 112 in the elongate member 104 partially through the opening 930 in the septum primum 610. An operator manipulates the occlusion device 520 and the elongate member 104 to implant it at the anatomical site of interest so ends 526a. 526b. 526c, and 526d of the occlusion device 520 span (cover) the hole 620 and the hole 930, thereby limiting the flow of blood between the first side 810 and the second side 820 through the hole 620 and hole 930. By way of example, as the ends 526a and 526b are extended from the opening 112 of the elongate member 104 onto the second side 820 of the septum, an operator may also withdraw the elongate member 104 thereby extending the ends 526c and 526d onto the first side of the septum.

By way of example, occlusion device 520 might be a septal occluder such as the CardioSEAL® medical device manufactured by NMT Medical, Inc. with offices in Boston, MA. FIG. 7C depicts the cross-section of the occlusion device 520 that has been implanted at the anatomical site of interest (defect in the septum) to restrict blood flow through the hole 620 and the hole 930.

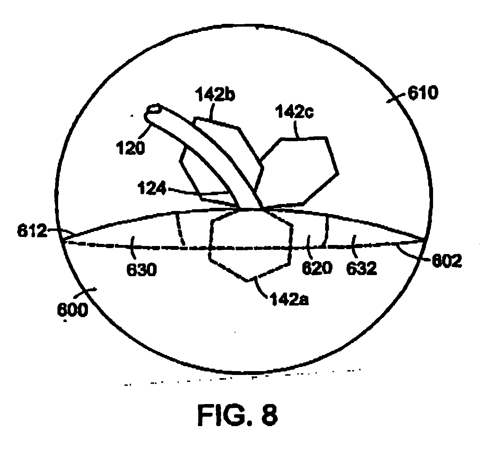

Illustrated in FIG. 8 is an alternative method of using the invention as viewed by, for example,an operator in a side view from a first side of a septum secundum, such as a side view from the first side 810 of the septum secundum 600 of FIG. 7B. In this method the illustrative septal puncture apparatus according to the invention includes the three flexible members 142a, 142b, and 142c connected to a distal end 124 of a delivery member 120. The flexible members 142a, 142b, and 142c are positioned by the operator in proximity to an opening 620 (e.g., a patent foramen ovale defect) between the septum primum 610 and the septum secundum 600. The septum primum 610 and the septum secundum 600 are partially overlapping cardiac tissue in which an edge 602 (shown with a dashed line as hidden) of the septum primum 610 overlaps with an edge 612 of the septum secundum 600. In this embodiment, the septum primum 610 and the septum secundum 600 are partially fused together in two regions 630 and 632, thereby defining the opening 620 between the septum primum 610 and the septum secundum 600.

Referring to FIG. 8, exemplary flexible member 142a is projected through the opening 620 by the operator such that it is, at least partially, located on the side of the septum secundum 600 opposite to the side of the atrial septum on which the flexible members 142b and 142c are positioned. The flexible members 142b and 142c are located adjacent the opening 620 and sized and shaped to be in contact with an opposing side of the septum primum 610. Accordingly, in this embodiment of the invention, at least one flexible member 142 is located on a first side of a tissue, and at least one flexible member 142 is located on an opposing, second side of the tissue. In this embodiment of the invention an operator may apply forces to the septum prumum 610 by pushing on the septum primum 610 with the flexible members 142b and 142c. By way of example, an operator may use this embodiment of the invention to locate the distal end 124 of the delivery member 120 in proximity to the opening 620, immobilize the septum primum 610 by pushing on the septum primum 610 with the flexible elastic members 142b and 142c, and then introduce a cutting member through a lumen of the delivery member 120 to create a hole through the septum primum 610.

Variations, modifications, and other implementations of what is described herein will occur to those of ordinary skill in the art without departing from the scope of the invention claimed.

## Claims

1. A surgical apparatus (100) for stabilizing the motion of a tissue wall comprising:
a first elongate member (104) comprising a longitudinal axis having a distal end, a proximal end, and a lumen (110) therein;
a delivery member (120) comprising a longitudinal axis having a distal end and a proximal end, wherein the delivery member is disposed within the lumen of the first elongate number;
a first flexible member (142'a) to contact a portion of tissue wall, disposed within the lumen of the elongate member, said first flexible member comprising a first end portion and a second end portion, the first end portion being connected to the distal end (124) of the delivery member and the second end portion being a free end, wherein when the first flexible member is located within the lumen of the elongate member, the first flexible member is oriented substantially parallel to the elongate member with the second end portion of the first flexible member directed distally; and
**characterized in that**:
when the first flexible member is partially extended out of the lumen of the elongate member by the delivery member (120), the second end portion of the first flexible member points in a proximal direction and is substantially parallel to the longitudinal axis of the elongate member.

2. The surgical apparatus of claim 1, wherein the delivery member further comprises a lumen (308).

3. The surgical apparatus of claim 2, wherein the delivery member further comprises a cutting member (300) disposed within the lumen.

4. The surgical apparatus of claim 3, wherein the cutting member is a needle.

5. The surgical apparatus of claim 1, further comprising a second flexible member (142'b) disposed within the lumen of the elongate member, said second flexible member comprising a first end portion and a second end portion, the first end portion being connected to the distal end of the delivery member and the second end portion being a free end, wherein when the second flexible member is located within the lumen of the elongate member, the second flexible member is oriented substantially parallel to the elongate member with the second end portion of the second flexible member directed distally, and wherein when the second flexible member is partially extended out of the lumen of the elongate member, the second end portion of the second flexible member points in a proximal direction and is substantially parallel to the longitudinal axis of the elongate member.

6. The surgical apparatus of claim 1 or 5, wherein the flexible member is pre-shaped such that the flexible member will assume a predetermined extended configuration when extended from the lumen of the elongated member to the extended position.

7. The surgical apparatus of claim 1 or 5, wherein the flexible member comprises a wire.

8. The surgical apparatus of claim 5, wherein the second end portion of the first flexible member and the second end portion of the second flexible member point substantially towards one another once the distal end of the delivery member extends beyond the distal end of the elongate member.

9. The surgical apparatus of claim 8, wherein said first flexible member and said second flexible member are substantially planar in shape, wherein a plane defined by said first or second flexible member defines a plurality of axes non-parallel to the longitudinal axis of the elongated member.

10. The surgical apparatus of claim 1, wherein a diameter of the first flexible member is larger at the free second end portion than at the connected first end portion.

## Patentansprüche

1. Chirurgisches Gerät (100) zur Stabilisierung der Bewegung einer Gewebewand, umfassend:
ein erstes längliches Bauteil (104), welches eine Längsachse aufweist, das ein distales Ende, ein proximales Ende und ein Lumen (110) aufweist;
ein Zufuhrbauteil (120), welches eine Längsachse aufweist, das ein distales Ende und ein proximales Ende aufweist, wobei das Zufuhrbauteil innerhalb des Lumens des ersten länglichen Bauteils angeordnet ist;
ein erstes flexibles Bauteil (142'a) zur Kontaktaufnahme mit einem Abschnitt der Gewebewand, das innerhalb des Lumens des länglichen Bauteils angeordnet ist, wobei das erste flexible Bauteil einen ersten Endabschnitt und einen zweiten Endabschnitt umfasst, wobei der erste Endabschnitt mit dem distalen Ende (124) des Zufuhrbauteils verbunden ist und es sich bei dem zweiten Endabschnitt um ein freies Ende handelt, wobei, wenn das erste flexible Bauteil innerhalb des Lumens des länglichen Bauteils angeordnet ist, das erste flexible Bauteil im Wesentlichen parallel zum länglichen Bauteil ausgerichtet ist, wobei der zweite Endabschnitt des ersten flexiblen Bauteils in die distale Richtung gerichtet ist; und
**dadurch gekennzeichnet ist, dass**:
wenn sich das erste flexible Bauteil durch das Zufuhrbauteil (120) teilweise aus dem Lumen des länglichen Bauteils erstreckt, der zweite Endabschnitt des ersten flexiblen Bauteils in eine proximale Richtung zeigt und im Wesentlichen parallel zur Längsachse des länglichen Bauteils verläuft.

2. Chirurgisches Gerät gemäß Anspruch 1, **dadurch gekennzeichnet, dass** das Zufuhrbauteil ferner ein Lumen (308) umfasst.

3. Chirurgisches Gerät gemäß Anspruch 2, **dadurch gekennzeichnet, dass** das Zufuhrbauteil ferner ein Schneidebauteil (300) umfasst, welches innerhalb des Lumens angeordnet ist.

4. Chirurgisches Gerät gemäß Anspruch 3, wobei das Schneidebauteil eine Nadel ist.

5. Chirurgisches Gerät gemäß Anspruch 1, ferner ein zweites flexibles Bauteil (142'b) umfassend, das innerhalb des Lumens des länglichen Bauteils angeordnet ist, wobei das zweite flexible Bauteil einen ersten Endabschnitt und einen zweiten Endabschnitt umfasst, wobei der erste Endabschnitt mit dem distalen Ende des Zufuhrbauteils verbunden ist und es sich bei dem zweiten Endabschnitt um ein freies Ende handelt, wobei, wenn das zweite flexible Bauteil innerhalb des Lumens des länglichen Bauteils angeordnet ist, das zweite flexible Bauteil im Wesentlichen parallel zum länglichen Bauteil ausgerichtet ist, wobei der zweite Endabschnitt des zweiten flexiblen Bauteils in die distale Richtung gerichtet ist, und wobei, wenn sich das zweite flexible Bauteil teilweise aus dem Lumen des länglichen Bauteils erstreckt, der zweite Endabschnitt des zweiten flexiblen Bauteils in eine proximale Richtung zeigt und im Wesentlichen parallel zur Längsachse des länglichen Bauteils ausgerichtet ist.

6. Chirurgisches Gerät gemäß Anspruch 1 oder 5, **dadurch gekennzeichnet, dass** das flexible Bauteil so vorgeformt ist, dass das flexible Bauteil eine zuvor festgelegte verlängerte Konfiguration einnimmt, wenn es sich aus dem Lumen des länglichen Bauteils in eine verlängerte Position erstreckt.

7. Chirurgisches Gerät gemäß Anspruch 1 oder 5, wobei das flexible Bauteil einen Draht umfasst.

8. Chirurgisches Gerät gemäß Anspruch 5, **dadurch gekennzeichnet, dass** der zweite Endabschnitt des ersten flexiblen Bauteils und der zweite Endabschnitt des zweiten flexiblen Bauteils im Wesentlichen zueinander hin zeigen, sobald das distale Ende des Zufuhrbauteils sich bis über das distale Ende des länglichen Bauteils erstreckt.

9. Chirurgisches Gerät gemäß Anspruch 8, **dadurch gekennzeichnet, dass** das erste flexible Bauteil und das zweite flexible Bauteil im Wesentlichen eine flache Form haben, wobei eine Ebene, die durch das erste oder zweite flexible Bauteil definiert wird, eine Vielzahl von Achsen definiert, die nicht-parallel zur Längsachse des länglichen Bauteils verlaufen.

10. Chirurgisches Gerät gemäß Anspruch 1, **dadurch gekennzeichnet, dass** ein Durchmesser des ersten flexiblen Bauteils am freien zweiten Endabschnitt größer ist als an dem verbundenen ersten Endabschnitt.

## Revendications

1. Instrument chirurgical (100) de stabilisation du mouvement d'une paroi tissulaire, comprenant :
un premier élément allongé (104) comprenant un axe longitudinal ayant une extrémité distale, une extrémité proximale et une lumière (110) à l'intérieur;
un élément d'avance (120) comprenant un axe longitudinal ayant une extrémité distale et une extrémité proximale, dans lequel l'élément d'avance est disposé dans la lumière du premier élément allongé ;
un premier élément flexible (142'a) destiné à entrer en contact avec une partie de paroi tissulaire, disposé dans la lumière de l'élément allongé, ledit premier élément flexible comprenant une première partie terminale et une seconde partie terminale, la première partie terminale étant reliée à l'extrémité distale (124) de l'élément d'avance et la seconde partie terminale étant une extrémité libre, dans lequel, lorsque le premier élément flexible est situé dans la lumière de l'élément allongé, le premier élément flexible est orienté sensiblement parallèlement à l'élément allongé, la seconde partie terminale du premier élément flexible étant dirigée selon la direction distale; et **caractérisé en ce que**:
lorsque le premier élément flexible est partiellement mis en extension hors de la lumière de l'élément allongé par l'élément d'avance (120), la seconde partie terminale du premier élément flexible pointe dans une direction proximale et est sensiblement parallèle à l'axe longitudinal de l'élément allongé.

2. Instrument chirurgical selon la revendication 1, dans lequel l'élément d'avance comprend en outre une lumière (308).

3. Instrument chirurgical selon la revendication 2, dans lequel l'élément d'avance comprend en outre un élément coupant (300) disposé dans la lumière.

4. Instrument chirurgical selon la revendication 3, dans lequel l'élément coupant est une aiguille.

5. Instrument chirurgical selon la revendication 1, comprenant en outre un second élément flexible (142'b) disposé dans la lumière de l'élément allongé, ledit second élément flexible comprenant une première partie terminale et une seconde partie terminale, la première partie terminale étant reliée à l'extrémité distale de l'élément d'avance et la seconde partie terminale étant une extrémité libre, dans lequel, lorsque le second élément flexible est situé dans la lumière de l'élément allongé, le second élément flexible est orienté sensiblement parallèlement à l'élément allongé et la seconde partie terminale du second élément flexible est alors dirigée selon la direction distale, et dans lequel, lorsque le second élément flexible est partiellement mis en extension hors de la lumière de l'élément allongé, la seconde partie terminale du second élément flexible pointe dans une direction proximale et est sensiblement parallèle à l'axe longitudinal de l'élément allongé.

6. Instrument chirurgical selon la revendication 1 ou 5, dans lequel l'élément flexible est préformé de manière que l'élément flexible prenne une configuration d'extension prédéterminée lorsqu'il est mis en extension hors de la lumière de l'élément allongé jusqu'à la position d'extension.

7. Instrument chirurgical selon la revendication 1 ou 5, dans lequel l'élément flexible comprend un fil.

8. Instrument chirurgical selon la revendication 5, dans lequel la seconde partie terminale du premier élément flexible et la seconde partie terminale du second élément flexible pointent sensiblement l'une vers l'autre lorsque l'extrémité distale de l'élément d'avance est en extension au-delà de l'extrémité distale de l'élément allongé.

9. Instrument chirurgical selon la revendication 8, dans lequel ledit premier élément flexible et ledit second élément flexible sont de forme sensiblement plane, dans lequel un plan défini par lesdits premier ou second éléments flexibles définit une pluralité d'axes non parallèles à l'axe longitudinal de l'élément allongé.

10. Instrument chirurgical selon la revendication 1, dans lequel un diamètre du premier élément flexible est plus grand à la seconde partie terminale libre qu'à la première partie terminale reliée.
